Europäisches Patentamt

European Patent Office

Office européen des brevets

(19)

(11) Publication number: 0 388 008

A1

## (12) EUROPEAN PATENT APPLICATION

(21) Application number: 90301495.9

(22) Date of filing: 13.02.90

(51) Int. Cl.5: **C12N 15/53, C12N 15/81,** **//(C12N15/81,C12R1:84)**

(30) Priority: 13.02.89 US 311517

(43) Date of publication of application:
19.09.90 Bulletin 90/38

(84) Designated Contracting States:
AT BE CH DE DK ES FR GB GR IT LI LU NL SE

(71) Applicant: THE SALK INSTITUTE
BIOTECHNOLOGY, INDUSTRIAL
ASSOCIATES
10280 North Torrey Pines Road
La Jolla, California 92037(US)

(72) Inventor: Craig, William Scot
5519 Honors Drive
San Diego, California 92122(US)
Inventor: Davis, Geneva Ruth
3083 East Fox Run
San Diego, California 92111(US)
Inventor: Holtz, Gregory Clyde
3083 East Fox RunWay
San Diego,California 92111(US)

(74) Representative: Allard, Susan Joyce et al
BOULT, WADE & TENNANT, 27 Furnival
Street
London EC4A 1PQ(GB)

(54) Production of superoxide dismutase in pichia pastoris yeast cells.

(57) The present invention provides compositions and methods for the production of the enzyme superoxide dismutase, and particularly human superoxide dismutase, by culturing P. pastoris that has been transformed to express the enzyme.

EP 0 388 008 A1

# PRODUCTION OF SUPEROXIDE DISMUTASE IN PICHIA PASTORIS YEAST CELLS

The present invention relates generally to advances in molecular biology and recombinant DNA technology.

More particularly, the present invention is directed to a process of recombinant DNA technology for producing polypeptides having superoxide dismutase (hereinafter also referred to as SOD) activity, in Pichia pastoris yeast cells. Pichia pastoris transformants containing in their genome at least one copy of a DNA sequence operably encoding the desired polypeptide under the regulation of a promoter region of a P. pastoris gene are cultured under conditions allowing the expression of the SOD gene product. The invention further relates to the P. pastoris transformants, DNA fragments and expression vectors used for their production and cultures containing same.

A variety of normal and pathological processes in aerobic cells lead to the production of tissue-damaging free radicals. It is believed that superoxide radical ($O_2.^-$), in many cases generated by the enzyme xanthine oxidase, is the primary mediator of such damages that may occur, for example, when tissue is reperfused after an organ transplant or after removal of a blood clot. Superoxide radical has also been implicated in the inflammatory response of phagocytes. Evidence for this involvement is derived from the observation that a family of enzymes capable of destroying superoxide (superoxide dismutases), can substantially reduce superfusion damage and possess anti-inflammatory properties.

Superoxide dismutases are enzymes that have either Cu(II) and Zn(II), or Mn(II) alone, or Fe(II) alone at the active site to catalyze the reaction:

$$O_2.^- + O_2.^- + 2H^+ \rightarrow H_2O_2 + O_2$$

The first protein that was shown in 1969 to possess superoxide dismutase activity [McCord et al., J. Biol. Chem. 244, 6049 (1969)] was isolated from erythrocytes in 1939 and was named erythrocuprein. Since then the genes of several members of the family have been cloned [Sherman et al., Proc. Natl. Acad. Sci. USA 80, 5465 (1983); Hallewell et al., Nucleic Acids Res. 13, 2017 (1985); Beck et al., Nucleic Acids Res. 15, 9076 (1987)] and the x-ray crystallographic structure has been determined for the bovine Cu/Zn enzyme [Richardson et al., Proc. Natl. Acad. Sci. USA 72, 1349 (1975)]. The amino acid sequence of human superoxide dismutase (hSOD), an eukaryotic cytoplasmic Cu/Zn enzyme, is described in Jabusch et al., Biochemistry 19, 2310 (1980). According to this, hSOD is a non-covalent dimer of identical subunits, each containing 153 amino acids. The amino terminus of the polypeptide chain is acetylated. The nucleic acid and predicted amino acid sequences of hSOD monomer, as reported in the European Patent Application Publication No. 233,244, are shown in Figure 1. The sequences reported in the literature may show several nucleotide differences but this seems to have no effect to the overall structure and properties of hSOD.

A comparison of the deduced amino acid sequence of mature human extracellular superoxide dismutase (EC-SOD) with those of Cu/Zn SODs from human, pig, cow, horse, swordfish, fruit fly, spinach, S. cerevisiae, and P. leiognathi is presented by Hjalmarsson et al., in Proc. Natl. Acad. Sci. USA 84, 6340 (1987).

The capability of superoxide dismutases to catalyse the destruction of superoxide radicals renders them useful in a variety of therapeutic applications. Bovine-derived SOD is currently marketed as an antiinflammatory human pharmaceutical or as a veterinary product, particularly for the treatment of inflamed tendons in horses. The potential pharmaceutical applications are even more numerous. In the endeavours to exploit the full therapeutic potential of superoxide dismutases, it may be advantageous to use the hSOD enzyme for human therapeutics in order to eliminate the adverse immunological responses possible with the antigenically distinct bovine enzyme.

Both human and veterinary applications of superoxide dismutases require large quantities of these materials that can be produced only by means of recombinant DNA technology.

The expression of Cu/Zn hSOD in E. coli under the regulation of the tacI promoter has been reported by Hallewell et al., Nucleic Acids Res. 13, 2017 (1985). In the polypeptide expressed in E. coli the N-terminal methionine is removed and, unlike in authentic hSOD, the N-terminal alanine group is not acetylated. Out of 500 clones examined, there were only about 25 that synthesized 5% or more of soluble cell protein as SOD. In an experiment that appears to be their best, about 15% of soluble cell protein was identified as SOD. The lack of N-terminal acetylation is disadvantageous. Where the naturally occurring form of a polypeptide, as is the case for hSOD, is N-acetylated, for therapeutic or diagnostical use the authentic, i.e. N-acetylated recombinant products are preferred. In this way immunogenicity can be eliminated or at least minimized when the recombinant product is administered to a human or animal host. Also, acetylated polypeptides are usually more stable and resistant to degradation by proteases.

Yeasts appeared to be good alternatives as hosts for the production of superoxide dismutases for a number of reasons. In the case of eukaryotic heterologous proteins, e.g., SODs, yeasts can offer clear advantages over bacteria. The intracellular environment of yeasts, being eukaryotes themselves, is more likely conducive to proper folding of the molecule. In addition, yeasts can generally be grown to higher cell densities than bacteria. Further, the initiator methionine is often processed by yeast; this is not often observed in bacteria.

The production of hSOD in S. cerevisiae (Baker's yeast) is disclosed in the European Patent Application No. 84111416.8, published April 24, 1985 under No. 0,138,111. In laboratory-scale experiments Cu/Zn hSOD was expressed in the order of about 10 to 30% of total cell protein, in N-acetylated, authentic form.

Using Saccharomyces cerevisiae strain 2150-2-3 leu⁻, Hallewell et al., Bio/Technology 5, 363 (1987) (inventors of the above-identified patent application) report the production of an N-acetylated, authentic form of Cu/Zn hSOD. Although according to the article hSOD has been expressed "at very high levels" under the regulation of the yeast glyceraldehyde phosphate dehydrogenase promoter, the authors do not provide any quantitative data as to the expression levels. Furthermore, the authors do not present data which shows that it is solely hSOD, and hot hSOD in combination with yeast SOD, which comprises the expression level. The hSOD levels are reported to be between 30% and 70% of total cell protein in stationary-phase yeast. Their results in exponentially growing cells were considerably worse so that hSOD was occasionally undetectable during exponential growth. This indicates that growing the cells to stationary phase is a necessity to obtain reasonable levels of expression. This, in turn, results in a lengthly fermentation procedure with no practical possibility of continuous mode.

Although S. cerevisiae is probably the most thoroughly investigated yeast, it is usually an imperfect candidate for the production of foreign proteins. In many instances problems have been encountered in attempts to move from the laboratory to a commercially useful scale. One source of this problem is that the promoters available for expression in S. cerevisiae are relatively weak and not well regulated. As a result, expression constructs normally must be placed on multi-copy plasmids to obtain an acceptable level of expression. This is true for the production of hSOD in S. cerevisiae as described by Hallewell et al. Supra. In fermentors operating at high cell density, selection for plasmid maintenance is lost, and plasmid distribution, copy number and stability become a problem.

To overcome these and other problems associated with S. cerevisiae, a yeast expression system based on Pichia pastoris has been developed. P. pastoris is a methylotrophic yeast; it has metabolic pathways that respond to, and regulate, methanol utilization. A key enzyme in the methanol pathway is alcohol oxidase, a protein encoded by two genes, alcohol oxidase I (AOX1) and alcohol oxidase II (AOX2). When P. pastoris cells are grown in the presence of methanol, the AOX1 and AOX2 genes are transcribed and a large amount of alcohol oxidase protein is made. The high level of AOX gene expression is mainly due to the AOX1 gene promoter that is activated in the presence of methanol and is highly expressed and tightly regulated (see e.g. European Patent Application No. 85113737.2 published October 30, 1984, under No. 183,071). A more complete description of a methanol-responsive P. pastoris expression system that places heterologous genes under the regulation of the P. pastoris AOX1 promoter is found in Cregg et al., Bio/Technology 5, 479 (1987).

Although P. pastoris has been used successfully for the production of several heterologous proteins, e.g., hepatitis B surface antigen (HBsAg) (Cregg et al., Supra) or tumor necrosis factor, endeavors to produce other heterologous gene products in Pichia have given mixed results. At our present level of understanding of the P. pastoris expression system, it is unpredictable whether a given gene can be expressed in this yeast, whether the expression levels will be satisfactory, or whether Pichia will tolerate the presence of the recombinant gene product in its cells.

The present invention is based on the utilization of the P. pastoris expression system for the production by recombinant DNA technology, of polypeptides having SOD activity. The process according to the invention is very efficient, producing SODs, in particular hSOD, at surprisingly high levels, and can be easily scaled up from shake-flask cultures to large fermenters without considerable changes in the fermentation conditions or loss in efficacy. Since double-copy strains provide considerably higher expression levels than strains that have integrated only a single copy of the expression cassette in their genome, the expression system according to the invention has great potentials in increasing the expression levels even further by introducing additional copies of the expression cassette into the yeast genome.

Pichia pastoris is a known industrial yeast strain that is capable of utilizing methanol as the sole carbon and energy source. According to our experiments, polypeptides having SOD activity, e.g., human SOD (hSOD), can be efficiently produced by integrating at least one copy of an expression cassette containing a DNA sequence encoding for the desired SOD polypeptide under the regulation of a promoter region of a P. pastoris gene into the genome of the host strain.

For gene replacement, a linear DNA fragment containing at least one copy of the expression cassette is inserted into a desired locus, for example, the AOX1 locus where it replaces the endogenous gene. If the AOX1 gene is disrupted due to the insertion of the expression unit, the still functional AOX2 gene supplies enough alcohol oxidase for slow growth on methanol. These strains are designated Mut⁻ for methanol utilization defective.

In the strains developed using the gene addition technique, the expression cassette contained on the transforming plasmid is added into the desired locus, e.g. the AOX1 or HIS4 locus without disrupting the endogenous gene. These strains are designated Mut⁺ and show wild-type growth on methanol.

Stable integration was accomplished both by gene replacement and by plasmid addition. In either case, the development of transformants through the use of integrating vectors, as opposed to those based on autonomous plasmids (like in case of S. cerevisiae), results in more productive recombinant strains.

In the production of SOD polypeptides, both Mut⁻ and Mut⁺ strains performed well but the Mut⁺ were found to be superior. The Mut⁺ strain containing two integrated copies of the SOD expression cassette in its genome produced hSOD in at least three-fold higher concentration than the, also unusually high, concentration produced by single copy strains.

This invention relates to a P. pastoris cell containing in its genome at least one copy of a DNA sequence operably encoding in P. pastoris a polypeptide having SOD activity under the regulation of a promoter region of a P. pastoris gene.

According to another aspect, this invention relates to DNA fragments comprising in the direction of transcription, a promoter region of a first P. pastoris gene, a sequence operably encoding in P. pastoris a polypeptide having SOD activity and a transcription termination segment of a second P. pastoris gene, said first and second P. pastoris genes being identical or different. The DNA fragment according to the invention may further comprise a selectable marker gene, and ends having sufficient homology with a target gene to effect integration of said DNA fragment therein. Alternatively, the DNA fragments can be contained within, or may be, circular plasmids, which may be linearized and will integrate at a site of homology between the host and the plasmid.

The present invention further concerns a process for producing a polypeptide having SOD activity, comprising growing P. pastoris transformants containing in their genome at least one copy of a DNA sequence operably encoding in P. pastoris a polypeptide having superoxide dismutase activity under the regulation of a promoter region of a P. pastoris gene under conditions allowing the expression of said DNA sequence in said P. pastoris strain.

The polypeptides having SOD activity are produced according to the invention in properly folded, authentic form, have the correct size and surface charge. The obtained polypeptides are fully functional to yield the expected specific activity.

The present invention is directed to the above aspects and all associated methods and means for accomplishing such. For example, the invention includes the technology requisite to suitable growth of the P. pastoris host cells, fermentation, and extraction of the SOD polypeptide gene product. The invention further includes methods and means for the removal or inactivation of the endogenous P. pastoris SOD gene. Replacement of this gene with a DNA sequence encoding human SOD, under the control of a promoter region of the endogenous P. pastoris SOD gene, further improves the production levels, and yields hSOD in high purity, without any accompanying endogenous Pichia SOD. Gene disruption and replacement can be carried out by methods known in the art.

P. pastoris is described as a model system of the covered use of a methylotrophic yeast host, primarily due to its unique expression characteristics. Other useful methylotrophic yeasts can be taken from four genera, namely Candida, Hanensula, Pichia and Torulopsis. Equivalent species from them may be used as hosts herein primarily based upon their demonstrated characterization of being supportable for growth and exploitation on methanol as a single carbon nutriment source. See, for example, Gleeson et al., Yeast 4, 1 (1988).

Figure 1 shows the nucleic acid and predicted amino acid sequences of hSOD.

In Figure 2, the results of certain hSOD fermentation experiments are set forth.

Figure 3 is an illustration of superoxide dismutase assays.

An expression system suitable for the production of polypeptides having SOD activity is provided.

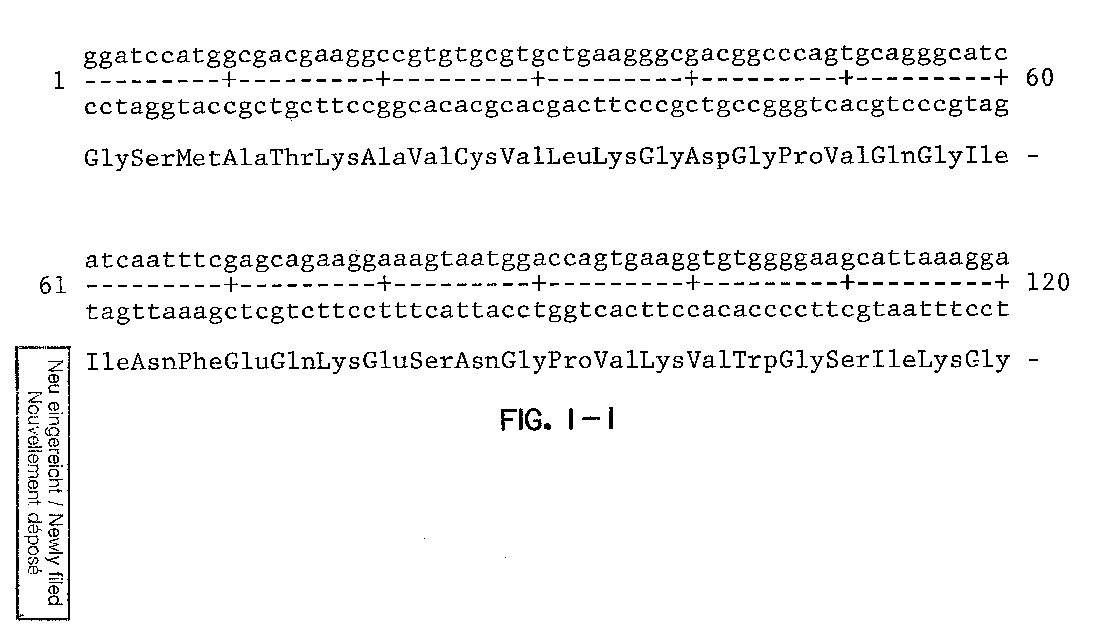

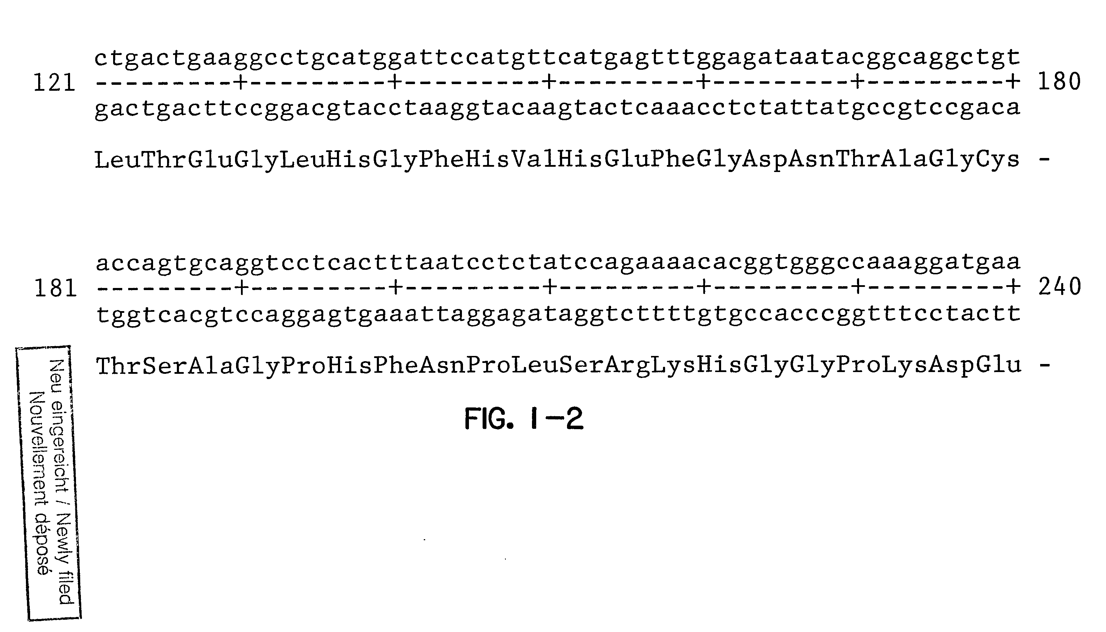

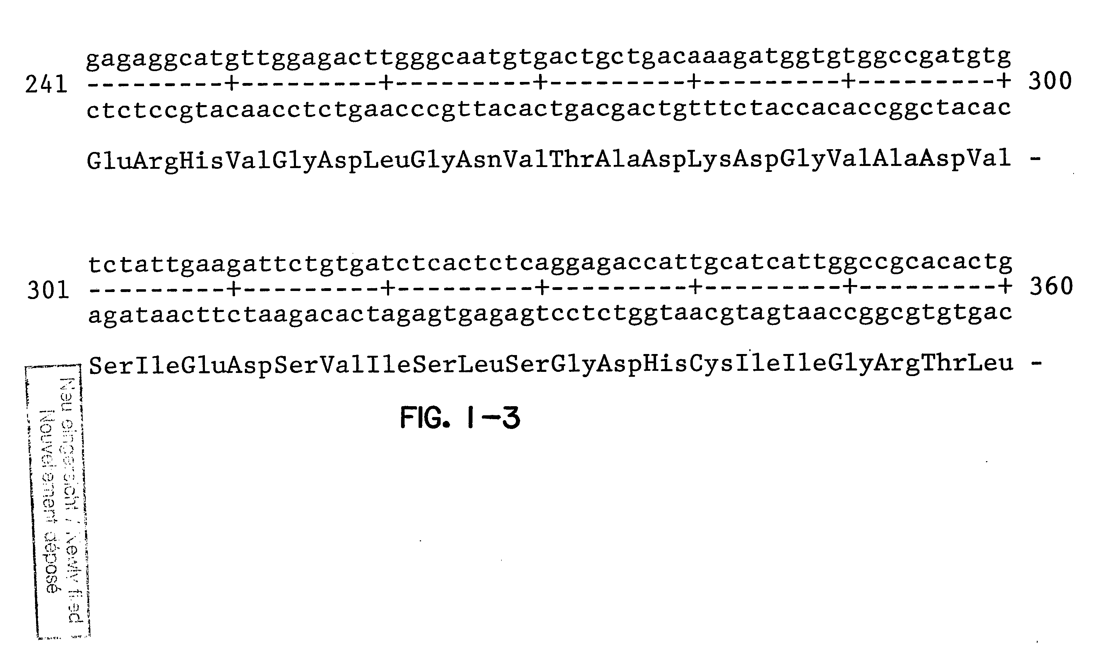

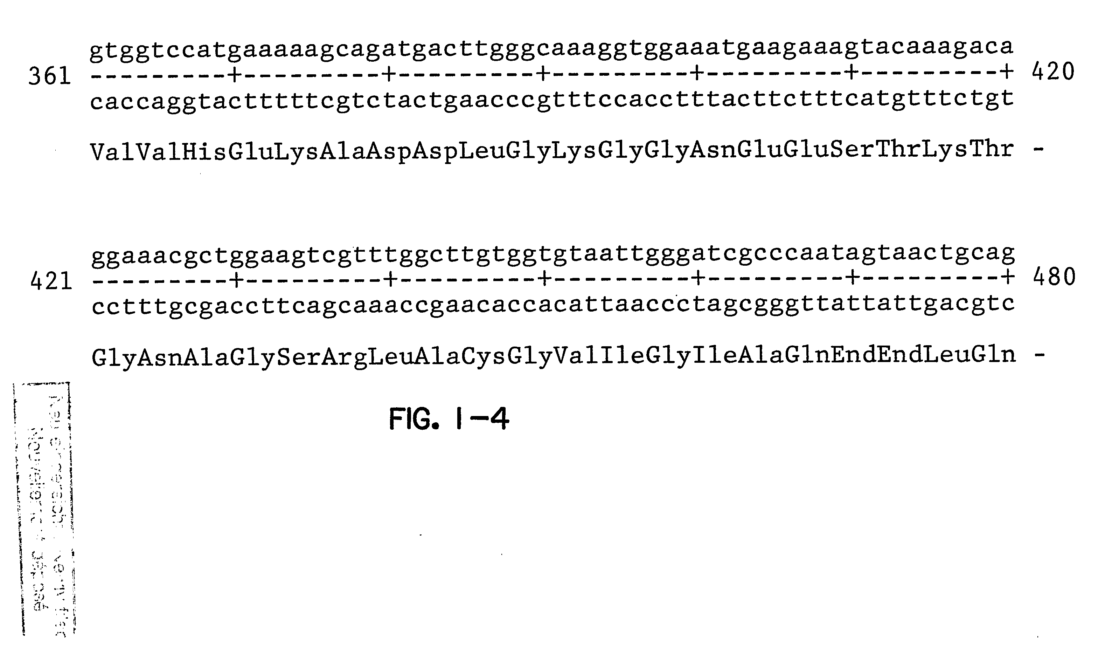

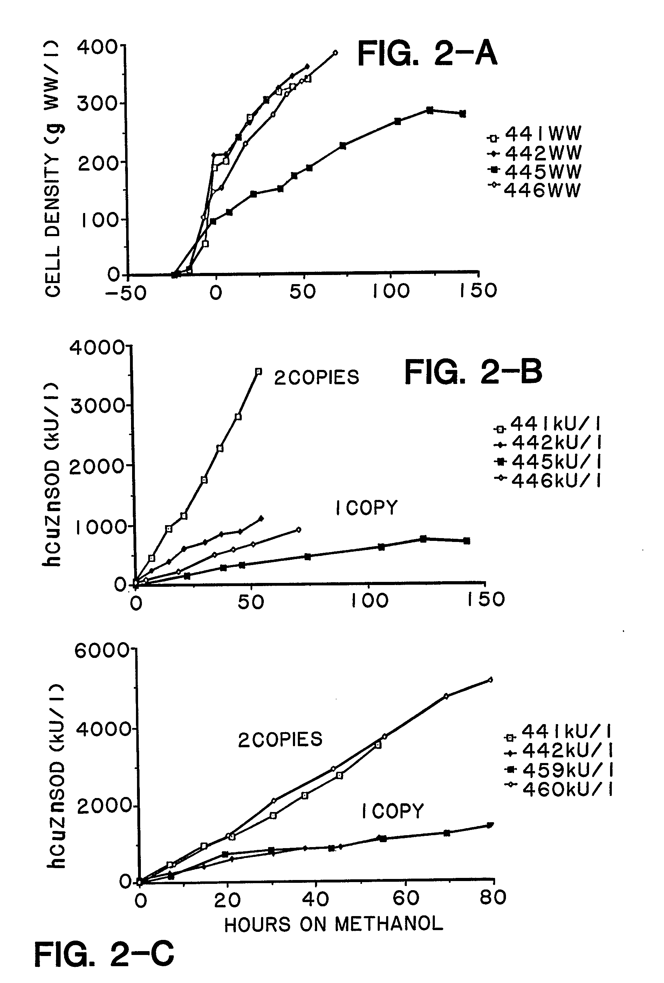

The term "polypeptide having SOD activity" is used to cover polypeptides of any origin, including plants, animals, such as mammals including human, their analogs and fragments exhibiting superoxide dismutase activity. For example, polypeptides in which one or both of the monomers, the amino acid sequence of which is illustrated in Figure 1 of the present application, or in Figure 2 of the Hjallmarson et al. article, Supra, lack one or more amino acids, or polypeptides containing additional amino acids, or polypeptides in which one or more amino acids in the depicted amino acid sequences are replaced by other amino acids are within the scope of the invention, as long as they exhibit SOD activity, in kind. The term is used to include dimer constructs containing two identical subunits (homodimers) as well as dimers consisting of two different subunits (heterodimers).

The term "SOD activity" and grammatical variations thereof, as used throughout the specification and claims refer to the activity exhibited by superoxide dismutases isolated from natural source in art-recognized bioassays, such as spectrophotometric assays and electrophoretic activity assays.

Since the substrate for SOD is the superoxide anion, that is an unstable free radical, there is no convenient method for directly detecting the enzyme's activity. The literature describes several indirect assays that monitor the steady state concentration of superoxide as it passes electrons to an indicator compound. Superoxide dismutase activity is measured by its effect as a competitor for the substrate, superoxide. These indirect assays must include a method for both generating and detecting superoxide. Generating superoxide may be accomplished enzymatically, e.g. with xanthine oxidase and xanthine, or photochemically, e.g. with riboflavin and tetramethylethylenediamine (TEMED). Alternatively, superoxide can be detected with electron acceptors such as ferricytochrome c, nitroblue tetrazolium (NBT), or through oxidation with hydroxylamine.

The standard SOD spectrophotometric assay uses xanthine oxidase acting on xanthine and molecular oxygen to generate superoxide, and the rate of cytochrome c reduction to detect it. Since superoxide cannot be generated in sufficient quantities to saturate the enzyme, classical methods for calculating the amount of enzyme are not useful. Instead, a unit of activity has been defined as the amount that causes a 50% inhibition of cytochrome c reduction under specific conditions [McCord et al., J. Biol. Chem. 244, 6049 (1969)].

A superior method for measuring SOD activity is an end point assay that is based on the oxidation of hydroxylamine to nitrite, which is then detected with an indicator dye [Elstner et al., Anal. Biochem. 70, 616 (1976)].

Alternatively, endogenous and heterologous SOD activities can be separated and assayed in P. pastoris in an acrylamide gel following non-denaturing electrophoresis. For this assay the most convenient superoxide generator and detector are, respectively, riboflavin/TEMED and nitroblue tetrazolium (NBT) [Beauchamp et al., Anal Biochem. 44, 276-287 (1971)].

Although it is sometimes difficult to duplicate test conditions exactly, and therefore results from different publications may vary, polypeptides showing the same activity, in kind as naturally-occurring superoxide dismutases in any of the above assays, or in other assays known in the art are all included within the scope of the present invention.

The amino acids, which occur in the various amino acid sequences referred to in the specification have their usual, three- and one-letter abbreviations, routinely used in the art, i.e.:

| Amino Acid | Abbreviation | |
|---|---|---|
| L-Alanine | Ala | A |
| L-Arginine | Arg | R |
| L-Asparagine | Asn | N |
| L-Aspartic Acid | Asp | D |
| L-Cysteine | Cys | C |
| L-Glutamine | Gln | Q |
| L-Glutamic Acid | Glu | E |
| Glycine | Gly | G |
| L-Histidine | His | H |
| L-Isoleucine | Ile | I |
| L-Leucine | Leu | L |
| L-Lysine | Lys | K |
| L-Methionine | Met | M |
| L-Phenylalanine | Phe | F |
| L-Proline | Pro | P |
| L-Serine | Ser | S |
| L-Threonine | Thr | T |
| L-Tryptophan | Trp | W |
| L-Tyrosine | Tyr | Y |
| L-Valine | Val | V |

According to the invention, polypeptides having SOD activity are produced by P. pastoris yeast cells containing in their genomes at least one copy of a DNA sequence operably encoding in P. pastoris a polypeptide having SOD activity under the regulation of a promoter region of a P. pastoris gene. The SOD-encoding DNA sequence is a gene encoding SOD, analogs and fragments thereof, as defined hereinabove. The gene may be obtained by known chemical synthesis techniques, enabled as the sequence has been published and is thus available, or by transcription of a messenger RNA (mRNA) corresponding to SOD to a complementary DNA (cDNA) and converting the single stranded cDNA obtained into a double stranded cDNA. The mRNA can be separated from any mammalian cell capable of producing polypeptides with SOD activity, such as from adult human liver cells.

The requisite DNA sequence can also be removed, for example, by restriction enzyme digest of known vectors harboring the gene. Examples of such vectors and the means for their preparation can be taken from the following prepublished documents: Hallewell et al., (1985) Supra (ptao5SOD plasmids), European Patent Application No. 88104880.5, published 28 September 1988, under No. 0,284,105 (pMSE-4 human manganese superoxide dismutase expression plasmid and related plasmids), European Patent Application No. 84111416.8, published 24 April 1985, under No. 0,138,111 (plasmids pC1/1GAPSOD and pc1/1GALAPSOD), etc.

The promoter region employed to drive the SOD gene expression is derived from a methanol-regulated alcohol oxidase gene of P. pastoris. As described hereinabove, P. pastoris is known to contain two functional alcohol oxidase genes: alcohol oxidase I (AOX1) and alcohol oxidase II (AOX2) genes. The coding portions of the two AOX genes are closely homologous at the DNA and predicted amino acid sequence levels and share common restriction sites. The proteins expressed from the two genes have similar enzymatic properties but the promoter of the AOX1 gene is more efficient and highly expressed, therefore, its use is preferred for SOD expression. The AOX1 gene, including its promoter, has been isolated and thoroughly characterized [Ellis et al., Mol. Cell. Biol. 5, 1111 (1985)].

An expression cassette, including the SOD encoding DNA together with the promoter region and a transcription termination segment is inserted into the host genome by means of a linear DNA fragment or a circular or linearized plasmid containing said DNA fragment. The transcription termination segment is a DNA segment taken from a P. pastoris protein-encoding gene, including a subsegment which encodes a polyadenylation signal and polyadenylation site in the transcript from the promoter used in the expression cassette, and another subsegment which provides a transcription termination signal for transcription from that promoter. The transcription termination segment may be derived from the same or different P. pastoris gene used as source of the promoter region.

The term "expression cassette" as used herein and throughout the specification and claims refers to a DNA sequence which includes sequences functional for expression.

The DNA fragments according to the invention may further comprise a selectable marker gene. For this purpose, any selectable marker gene functional in P. pastoris may be employed, i.e., any gene which confers a phenotype upon P. pastoris cells thereby allowing them to be identified and selectively grown from among a vast majority of untransformed cells. Suitable selectable marker genes include, for example, selectable marker systems composed of an auxotrophic mutant P. pastoris host strain and a wild-type biosynthetic gene which complements the host's defect. For transformation of his4⁻ P. pastoris strains, for example, the S. cerevisiae or P. pastoris HIS4 gene, or for transformation arg4⁻ mutants the S. cerevisiae ARG4 gene or the P. pastoris ARG4 gene, may be employed.

If the yeast host is transformed with a linear DNA fragment containing the SOD gene under the regulation of a promoter region of a P. pastoris gene, the expression cassette is integrated into the host genome by any of the gene replacement techniques known in the art, such as by one-step gene replacement [see e.g., Rothstein, Methods Enzymol. 101, 202 (1983); Cregg et al., Bio/Technology 5, 479 (1987)] or by two-step gene replacement methods [see e.g., Scherer and Davis, Proc. Natl. Acad. Sci. USA, 76, 4951 (1979)]. The linear DNA fragment is directed to the desired locus, i.e., to the target gene to be disrupted by means of flanking DNA sequences having sufficient homology with the target gene to effect integration of the DNA fragment therein. One-step gene disruptions are usually successful if the DNA to be introduced has as little as 0.2 kb homology with the fragment locus of the target gene; it is however, preferable to maximize the degree of homology for efficiency.

If the DNA fragment according to the invention is contained within or is an expression vector, e.g., a circular plasmid, one or more copies of the plasmid can be integrated at the same or different loci, by addition. Linearization of the plasmid by means of a suitable restriction endonuclease facilitates integration.

The term "expression vector" includes vectors capable of expressing DNA sequences contained therein, where such sequences are in operational association with other sequences capable of effecting their expression, i.e. promoter sequences. In general, expression vectors usually used in recombinant DNA technology are often in the form of "plasmids", i.e. circular, double-stranded DNA loops which, in their vector form, are not bound to the chromosome. In the present specification the terms "vector" and "plasmid" are used interchangeably. However, the invention is intended to include other forms of expression vectors as well, which function equivalently.

In the DNA fragments according to the invention the segments of the expression cassette are "in operational association". The DNA sequence encoding polypeptides having SOD activity are positioned and oriented functionally with respect to the promoter and the transcription terminator, so that the polypeptide encoding segment is transcribed, under regulation of the promoter region, into a transcript capable of providing upon translation the desired polypeptide in P. pastoris. Appropriate reading frame positioning and orientation of the various segments of the expression cassette are within the knowledge of persons of ordinary skill in the art; further details are given in the Examples.

The DNA fragments provided by the present invention may include sequences allowing for their replication and selection in bacteria, especially E. coli. In this way, large quantities of the DNA fragment can be produced by replication in bacteria.

Methods of transforming Pichia pastoris as well as methods applicable for culturing P. pastoris cells containing in their genome a gene for a heterologous protein are known generally in the art.

According to the invention, the expression cassettes are transformed into the P. pastoris cells either by the spheroplast technique, described by Cregg et al., Mol. Cell. Biol. 5, 3376 (1985) or by the whole-cell lithium chloride yeast transformation system [Ito et al., Agric. Biol. Chem. 48, 341 (1984)], with minor modification necessary for adaptation to P. pastoris. Although the whole-cell lithium chloride method is more convenient in that it does not require the generation and maintenance of spheroplasts, for the purpose of the present invention the spheroplast method is preferred, primarily since it yields a greater number of transformants.

Positive transformants are characterized by Southern blot analysis [Maniatis et al., Molecular Cloning: A Laboratory Manual, Cold Spring Harbor Laboratory Press, Cold Spring Harbor, New York, USA (1982)] for the site of DNA integration and number of integrants, and Northern blots [Maniatis, Op. Cit., R.S. Zitomer and B.D. Hall, J. Biol. Chem, 251, 6320 (1976)] for methanol-responsive SOD gene transcription.

Transformed strains, which are of the desired phenotype and genotype are grown in fermentors. For the large-scale production of recombinant DNA-based products in P. pastoris a two-stage, high cell-density, batch fermentation system is normally employed. In the first, or growth stage, expression hosts are cultured in defined minimal medium with glycerol as carbon source. On this carbon source, heterologous gene expression is completely repressed, which allows the generation of cell mass in the absence of heterologous protein expression. Subsequent to the depletion of the repressing carbon source, methanol is added, initiating the expression of the desired heterologous protein. This second stage is the so-called production stage.

The term "culture" means a propagation of cells in a medium conductive to their growth, and all subcultures thereof. The term "subculture" refers to a culture of cells grown from cells of another culture (source culture), or any subculture of the source culture, regardless of the number of subculturings which have been performed between the subculture of interest and the source culture.

In the fermentation experiments the following major medium components were employed:

| A. 10X BASAL SALTS | |
|---|---|
| Chemical | Grams/liter |
| Phosphoric Acid, 85% | 42.0 mls |
| Calcium Sulfate$\cdot$2H$_2$O | 1.8 |
| Potassium Sulfate | 28.6 |
| Magnesium Sulfate$\cdot$7H$_2$O | 23.4 |
| Potassium Hydroxide | 6.5 |

| B. IM$_1$ TRACE SALTS SOLUTION | |
|---|---|
| Chemical | Grams/liter |
| Cupric Sulfate$\cdot$5H$_2$O | 0.06 |
| Potassium Iodide | 0.08 |
| Manganese Sulfate$\cdot$H$_2$O | 0.30 |
| Sodium Molybdate | 0.20 |
| Boric Acid | 0.02 |
| Zinc Sulfate$\cdot$H$_2$O | 2.00 |
| Ferric Chloride$\cdot$H$_2$O | 4.8 |
| Sulfuric Acid | 5.00 ml/liter |

| C. YTM$_4$ TRACE SOLUTION + BIOTIN | |
|---|---|
| Chemical | Grams/liter |
| Ferrous Sulfate$\bullet$7H$_2$O | 65.0 |
| Copper Sulfate$\bullet$5H$_2$O | 6.0 |
| Zinc Sulfate$\cdot$7H$_2$O | 20.0 |
| Manganese Sulfate$\cdot$H$_2$O | 3.0 |
| Sulfuric Acid | 5.0 mls |
| Biotin | 0.1 |

According to a preferred embodiment of the invention, the heterologous protein expression system used for the production of polypeptides having SOD activity utilizes the promoter derived from the methanol-regulated AOX1 gene of P. pastoris, which is very efficiently expressed and tightly regulated. This gene is the source of the transcription termination segment as well.

The host cells to be transformed with the expression cassette are P. pastoris cells having at least one mutation that can be complemented with a marker gene present on a transforming DNA fragment. Preferably his4⁻ (GS115) auxotrophic mutant P. pastoris strains are employed.

The expression cassette containing a single copy of the SOD gene under the regulation of the AOX1 promoter and transcription terminator is placed in a pBR322-based Pichia expression vector, which also includes a selectable marker gene, such as HIS4 gene if the host P. pastoris strain is a his4⁻ auxotrophic mutant.

Preferably, the expression cassette is integrated into the host genome by transformation of the host with an uncut circular plasmid comprising the cassette. The integration is by addition at a locus or loci having homology with one or more sequences present on the transformation vector, e.g. 5′AOX1, 3′AOX1, HIS4, and will leave the AOX1 gene intact. In this way Mut⁺ strains are obtained that show wild-type growth on methanol. A Mut⁺ strain containing two integrated copies of the expression cassette was found to be superior, yielding considerably higher hSOD concentration than single-copy strains. It is possible to introduce additional copies of the expression cassette into the host strain by using a plasmid containing two or more SOD gene expression cassettes, preferably linked in tandem, and thereby increase productivity even further.

Alternatively, the expression cassette can be integrated into the host genome after digesting the expression vector with an appropriate enzyme yielding a linear DNA fragment with ends homologous to the AOX1 locus, by a one-step gene replacement technique. As a result of gene replacement, Mut⁻ strains are obtained. The still functional AOX2 gene supplies enough alcohol oxidase for slow growth on methanol.

In either case, the development of transformants through the use of integrating vectors, as opposed to those based on autonomous plasmids, results in more stable, productive recombinant strains.

The transformants in which the expression cassette has integrated into and disrupted the AOX1 locus by site-directed recombination can be selected by their his4[+] phenotype and by their decreased ability to utilize methanol (Mut[-]). Southern blot hybridization verified that the complete expression cassette has integrated at the AOX1 locus. Positive transformants are characterized by Southern analysis for the site of DNA integration and by Northern analysis for methanol-responsive SOD gene transcription. P. pastoris strains which have integrated one or multiple copies of the expression cassette at a desired site are identified by Southern blot hybridization.

P. pastoris transformants which are identified to have the desired genotype and phenotype are grown in fermentors. Typically a two-step production process is used. Initially, cells are grown on a repressing carbon source, preferably glycerol. In this stage the cell mass is generated in absence of expression. After exhaustion of glycerol, methanol alone (methanol fed-batch mode) or glycerol and methanol (mixed-substrate fed-batch mode) are added in the fermentor, resulting in the expression of the SOD gene driven by the A0X1 promoter. Mut[+] strains are preferably grown using a methanol-fed-batch procedure in which residual methanol is limited. Mut[-] strains, on the other hand, are preferably grown using either the methanol-fed batch or mixed-substrate fed-batch procedure in which residual methanol is kept in excess.

Polypeptides having SOD activity are produced in P. pastoris intracellularly and are usually extracted from Pichia cells lysed with glass beads in the presence of simple buffers known in the art. Typically, a 50 mM sodium or potassium phosphate buffer containing 0.1 mM EDTA at pH 7.8 is used, and recovery, after corrections for pellet volume, is at least 95% when measured by Western blots or activity assay. The small amount of the SOD product remaining in the pellet can be extracted with a strong detergent, such as sodium dodecyl sulfate (SDS).

The invention is further illustrated by the following non-limiting examples.

## Example 1

### Construction of hSOD expression vector, pSOD104

The expression vector construction disclosed in the present invention was performed using standard procedures, as described, for example in Maniatis et al., Molecular cloning: A Laboratory Manual, Cold Spring Harbor Laboratory Press, Code Spring Harbor, New York, USA (1982) and Davis et al. Basic Methods in Molecular Biology, Elsevier Science Publishing, Inc., New York (1986).

The human SOD gene (see Fig. 1) was received as a BamHI/PstI fragment in plasmid pEMBL8, as well as in an expression plasmid, pKK223-3, transformed into E. coli JM 105 cells, but can also be obtained from any of the prior published vectors, as set forth Supra.

MC1061 E. coli cells (that are widely available) were transformed with the pEMBL8 plasmid and amp[R] colonies were selected. Plasmid was prepared from the amp[R] cells and digested with BamHI and PstI. The correct plasmid contained a 480 bp BamHI/PstI insert comprised of the hSOD gene. This colony was used to prepare new plasmid DNA, the plasmid was digested with BamHI and PstI, and 50 ng of the 480 bp fragment (isolated on a 0.8% agarose gel) were ligated into 10 ng of BamHI-PstI-cut, phosphatase-treated M13mp19 plasmid [New England Biolabs] for sequencing and mutagenesis. JM103 cells (that are widely available) were transformed with the ligation mix and white plaques were selected. Plasmid was prepared from the white plaques and digested with BamHI and PstI. A 480 bp band was indicative of the correct plasmid, that was called pSOD101. The band thought to include the hSOD coding sequence was sequenced completely in one direction, and the sequence obtained agreed with that of the authentic hSOD gene (Fig. 1).

An EcoRI site was then added to the 3' end of the hSOD coding sequence immediately following the translation termination codon TAG. Site-directed mutagenesis was accomplished by the method of Zoller and Smith, Meth. Enzymol. 100, 468 (1983). The mutagenizing oligonucleotide was of sequence:
5'-GGA TCG CCC AAT AGG AAT TCC AGG CAT GCA AGC TT-3'
The screening oligonucleotide was of sequence:
5'-AAT AGG AAT TCC AGG CA-3'
A mini-template prep was performed on the positives from the screening and JM103 cells were transformed with the prep. White plaques were screened a second time with the screening oligonucleotide, and positives from this round of screening were used to prepare template for sequencing. Sequencing was carried out by following the Sanger dideoxy method Sanger et al. PNAS 74:5463 (1977) using the universal primer of sequence GTA AAA CGA CGG CCA GT.

A plasmid was identified as having the correctly modified 3′ end, and was called pSOD102.

The 5′ end of the hSOD insert was modified to add an EcoRI site immediately prior to the initiator ATG codon. The mutagenesis was accomplished by the same method as the 3′ mutagenesis except the starting plasmid was pSOD102; the mutagenizing oligonucleotide was of sequence:

5′-TCG AGC TCG GTA CCC AAG AAT TCA TGG CGA CGA AGG CC-3′

The screening oligonucleoide was of sequence:

5′-TAC CCA AGA ATT CAT G-3′

and the sequencing oligonucleotide was the universal primer.

Sequencing confirmed the correctly mutagenized 5′-end of the plasmid. The plasmid having EcoRI sites at the 5′- and 3′-ends of the hSOD insert was called pSOD103.

Plasmid pSOD103 was digested with EcoRI and the about 480 bp fragment was isolated on a 0.8% agarose gel. 50 ng of the hSOD fragment were ligated to 10 ng of EcoRI- cut, phosphatase-treated pAO804. (The preparation of plasmid pAO804 is described hereinbelow.) The ligation was transformed into MC1061 cells and amp$^R$ colonies were selected. Correct plasmid was identified by screening lifts with an oligonucleotide of the sequence:

$$\text{5'-AAA CGA GGA ATT CAT GGC GA-3'}$$

```
        |          |
————————|——————————|————————
AOX1      ECORI      SOD
```

The correct plasmid was called pSOD104.

Plasmid pAO804 employed in the above procedure was constructed as follows:

Plasmid pBR322 was modified as follows to eliminate the EcoRI site and insert a BglII site into the PvuII site:

pBR322 was digested with EcoRI, the protruding ends were filled in with Klenow Fragment of E. coli DNA polymerase I, and the resulting DNA was recircularized using T4 ligase. The recircularized DNA was used to transform E. coli MC1061 to ampicillin-resistance and transformants were screened for having a plasmid of about 4.37 kpb in size without an EcoRI site. One such transformant was selected and cultured to yield a plasmid, designated pBR322ΔRI, which is pBR322 with the EcoRI site replaced with the sequence:

5′-GAATTAATTC-3′
3′-CTTAATTAAG-5′.

pBR322ΔRI was digested with PvuII and the linker,

of sequence
5′-CAGATCTG-3′
3′-GTCTAGAC-5′

was ligated to the resulting blunt ends employing T4 ligase. the resulting DNAs were recircularized, also with T4 ligase, and then digested with BglII and again recircularized using T4 ligase to eliminate multiple BglII sites due to ligation of more than one linker to the PvuII-cleaved pBR322ΔRI. The DNAs, treated to eliminate multiple BglII sites, were used to transform E. coli MC1061 to ampicillin-resistance. Transformants were screened for a plasmid of about 4.38 kbp with a BglII site. One such transformant was selected and cultured to yield a plasmid, designated pBR322ΔRIBGL, for further work. Plasmid pBR322ΔRIBGL is the same as pBR322ΔRI except that pBR322ΔRIBGL has the sequence

5′-CAGCAGATCTGCTG-3′
3′-GTCGTCTAGACGAC-5′

in place of the PvuII site in pBR322ΔRI.

pBR322ΔRIBGL was digested with a SalI and BglII and the large fragment (approximately 2.97 kbp) was isolated. Plasmid pBSAGI5I, which is described in European Patent Application Publication No. 0,226,752, was digested completely with BglII and XhoI and an approximately 850 bp fragment from a region of the P. pastoris AOX1 locus downstream from the AOX1 gene transcription terminator (relative to the direction of transcription from the AOX1 promoter) was isolated. The BglII-XhoI fragment from pBSAGI5I and the approximately 2.97 kbp, SalI-BglII fragment from pBR322ΔRIBGL were combined and subjected to ligation with T4 ligase. The ligation mixture was used to transform E. coli MC1061 to ampicillin-resistance and transformants were screened for a plasmid of the expected size (approximately 3.8 kbp) with a BglII site. This plasmid was designated pAO801. The overhanging end of the SalI site from the pBR322ΔRIBGL fragment was ligated to the overhanging end of the XhoI site on the 850 bp pBSAGI5I fragment and, in the process, both the SalI site and the XhoI site in pA0801 were eliminated.

pBSAGI5I was then digested with ClaI and the approximately 2.0 kbp fragment was isolated. The 2.0 kbp fragment has an approximately 1.0-kbp segment which comprises the P. pastoris AOX1 promoter and transcription initiation site, an approximately 700 bp segment encoding the hepatitis B virus surface antigen ("HBsAg") and an approximately 300 bp segment which comprises the P. pastoris AOX1 gene polyadenylation signal and site-encoding segments and transcription terminator. The HBsAg coding segment of the 2.0 kbp fragment is terminated, at the end adjacent the 1.0 kbp segment with the AOX1 promoter, with an EcoRI site and, at the end adjacent the 300 bp segment with the AOX1 transcription terminator with a StuI site, and has its subsegment which codes for HBsAg oriented and positioned, with respect to the 1.0 kbp promoter-containing and 300 bp transcription terminator-containing segments, operatively for expression of the HBsAg upon transcription from the AOX1 promoter. the EcoRI site joining the promoter segment to the HBsAg coding segment occurs just upstream (with respect to the direction of transcription from the AOX1 promoter) from the translation initiation signal-encoding triplet of the AOX1 promoter.

For more details on the promoter and terminator segments of the 2.0 kbp, ClaI-site-terminated fragment of pBSAGI5I, see European Patent Application Publication No. 226,846 and Ellis et al., Mol. Cell Biol. 5, 1111 (1985).

Plasmid pA0801 was cut with ClaI and combined for ligation using T4 ligase with the approximately 2.0 kbp ClaI-site-terminated fragment from pBSAGI5I. The ligation mixture was used to transform E. coli MC1061 to ampicillin resistance, and transformants were screened for a plasmid of the expected size (approximately 5.8 kbp) which, on digestion with ClaI and BglII, yielded fragments of about 2.32 kbp (with the origin of replication and ampicillin-resistance gene from pBR322) and about 1.9 kbp, 1.48 kbp, and 100 bp. On digestion with BglII and EcoRI, the plasmid yielded an approximately 2.48 kbp fragment with the 300 bp terminator segment from the AOX1 gene and the HBsAg coding segment, a fragment of about 900 bp containing the segment from upstream of the AOX1 protein encoding segment of the AOX1 gene in the AOX1 locus, and a fragment of about 2.42 kbp containing the origin of replication and ampicillin resistance gene from pBR322 and an approximately 100 bp ClaI-BglII segment of the AOX1 locus (further upstream from the AOX1-encoding segment than the first mentioned 900 bp EcoRI-BglII segment). Such a plasmid had the ClaI fragment from pBSAGI5I in the desired orientation, in the opposite undesired orientation, there would be EcoRI-BglII fragments of about 3.3 kbp, 2.38 kbp and 900 bp.

One of the transformants harboring the desired plasmid, designated pA0802, was selected for further work and was cultured to yield that plasmid. The desired orientation of the ClaI fragment from pBSAGI5I in pA0802 had the AOX1 promoter region oriented correctly to lead to the correct integration into the P. pastoris genome at the AOX1 locus of linearized plasmid made by cutting at the BglII site at the terminus of the 800 bp fragment from downstream of the AOX1 gene in the AOX1 locus (terminator region) and the BglII site at the beginning of the promoter region.

pA0802 was then treated to remove the HBsAg coding segment terminated with an EcoRI site and a StuI site. The plasmid was digested with StuI and a linker of sequence:

5′-GGAATTCC-3′
3′-CCTTAAGG-5′

was ligated to the blunt ends using T4 ligase. The mixture was then treated with EcoRI and again subjected to ligation using T4 ligase. The ligation mixture was then used to transform E. coli MC1061 to ampicillin resistance and transformants were screened for a plasmid of the expected size (5.1 kbp) with EcoRI-BglII fragments of about 1.78 kbp, 900 bp, and 2.42 kbp and BglII-ClaI fragment of about 100 bp, 2.32 kbp, 1.48 kbp, and 1.2 kbp. This plasmid was designated pA0803. A transformant with the desired plasmid was selected for further work and was cultured to yield pA0803.

Plasmid pA0804 was then made form pA0803 by inserting, into the BamHI site from pBR322 in pA0803, an approximately 2.75 kbp BglII fragment from the P. pastoris HIS4 gene. See, e.g., Cregg et al., Mol. Cell. Biol. 5, 3376 (1985) and European Patent Application Publication Nos 0,180,899 and 0,188,677. pA0803 was digested with BamHI and combined with the HIS4 gene-containing BglII site-terminated fragment and the mixture subjected to ligation using T4 ligase. The ligation mixture was used to transform E. coli MC1061 to ampicillin-resistance and transformants were screened for a plasmid of the expected size (7.85 kbp), which is cut by SalI. One such transformant was selected for further work, and the plasmid it harbors was designated pA0804.

pA0804 has one SalI-ClaI fragment of about 1.5 kbp and another of abut 5.0 kbp and a ClaI-ClaI fragment of 1.3 kbp; this indicates that the direction of transcription of the HIS4 gene in the plasmid is the same as the direction of transcription of the ampicillin resistance gene and opposite the direction of transcription from the AOX1 promoter.

The orientation of the HIS4 gene is pA0804 is not critical to the function of the plasmid or of its derivatives with cDNA coding segments inserted at the EcoRI site between the AOX1 promoter and terminator segments. Thus, a plasmid with the HIS4 gene in the orientation opposite that of the HIS4 gene in pA0804 would also be effective for use in accordance with the present invention.

.

Example 2

Development of hSOD-expressing P. pastoris strains G-SOD104C and G + SOD104C

Plasmid pSOD104, constructed as described in Example 1, was used to develop both Mut[+] and Mut[-] strains of P. pastoris. The host strain was the histidine-requiring auxotroph GS115 (American Type Culture Collection, Rockville, Masryland, USA (ATCC) Accession No. 20864, deposited under the terms of the Budapest Treaty). Transformation was carried out by the spheroplast method described by Cregg et al., Mol. Cell. Biol. 5, 3376 (1985).

To develop Mut[+] strains, undigested pSOD104 was transformed into GS115 and His[+] cells were selected. Nine of the His[+] prototrophs were examined by Southern hybridization analyses. The chromosomal DNAs were digested with EcoRI and probed with pAO803, containing AOX1 5′ and 3′ regions, or pYM4 containing the Pichia HIS4 gene. A BglII digestion was also performed and probed with an oligonucleotide of sequence:

5′-AAC TCA TGA ACA TGG AAT CCA TGC AGG CCT-3′

which is homologous to a segment of the hSOD gene. The results of the Southern identified three classes of Mut[+] transformants which are are summarized below:

| Strain Name | Copy Number | Site of Integration |
|---|---|---|
| G + SOD104C1,4,5,7,8 | one | AOX1 |
| G + SOD104C2,9,10 | one | HIS4 |
| G + SOD104C3 | two | HIS4 and AOX1 |

Three of these transformants, G + SOD104C1 (one copy at AOX1 locus), G + SOD104C3 (one copy at both AOX1 and HIS4 loci), and G + SOD104C10 (one copy at HIS4 locus), were grown up in shake flasks along with the negative control, untransformed GS115. Cells were grown in glycerol as described hereinafter for the Mut[-] strains, washed, and seeded at an $OD_{600}$ of about 0.05 into 1X buffered YBN (0.67% Yeast Nitrogen Base) containing 1% methanol. Aliquots, each containing 50 ODs, were removed after 20, 23, and 26 hours of growth on methanol for analysis of the hSOD and mRNA levels.

RNA was analyzed by Northern blots. Approximately 5 μg of total RNA from each time point was denatured, separated on a formaldehyde-aragose gel, and transferred to nitrocellulose. The blot was probed with an oligonucleotide 30 bases in length (same oligo sequence as above) and homologous to hSOD. The Northern hybridization assay indicated that there was a significant amount of hSOD mRNA at all time points. Transformant G + SOD1014C3, which contains two copies of the expression vector, appeared to have several-fold more hSOD mRNA than the other two transformants. The control, GS115, showed no mRNA homologous to the oligonucleotide probe. All three of the transformants appeared to possess hSOD activity using electrophoretic separation and assay (described in Example 4).

To develop Mut[-] strains, a BglII digest of plasmid pSOD104 was transformed into GS115 cells, and His[+] cells were selected. The His[+] prototrophs were then screened for their Mut phenotype as follows:

Screening for Mut phenotype was accomplished by plating His[+] transformants on minimal glycerol (2%) master plates to obtain colonies originating from single cells. After two days incubation at 30°C, the masters were replica-plated to minimal glycerol plates and plates containing no carbon source to which methanol was added in vapor phase. This was done by adding a drop, approximately 200 μl, of methanol to the underside of the top of the cover of the petri dish holding the plate. The plates were incubated at 30°C for two days with additional methanol added in vapor phase every day. Colonies showing visible growth were scored as Mut[+] and those with no visible growth were scored as Mut[-].

Approximately 12% of the transformants were slow growers, indicative of the disruption of the AOX1 locus. Twenty two of these were compared with the control strain, G-PAO804, for growth rates on methanol.

Strain G-PAO804 was developed by disrupting the AOX1 locus with a BglII digest of the base plasmid pAO804. It displays the expected Mut⁻ phenotype but it does not express a recombinant gene product. All hSOD Mut⁻ transformants appeared to grow at approximately the same rate as G-PAO804. A slower growth rate can be indicative of toxicity to the cells from the heterologous gene product.

Nine of the His⁺ Mut⁻ cells were analyzed by Southern blots, as described hereinabove. All nine strains were shown to have integrated one copy of the expression cassette at the AOX1 locus, thus disrupting the gene. These strains were named G-SOD104C1 through G-SOD104C9.

Three of these, as well as the control strain G-PAO804, were grown in shake flasks to analyze the level and methanol-regulated nature of hSOD-specific mRNA. These cells were grown to stationary phase in 1X buffered YBN (0.67% Yeast Nitrogen Base) containing 5% glycerol, washed in water, and seeded at an $OD_{600}$ of approximately 1.0 into 1x YBN containing 1% methanol. Aliquots, each containing 100 ODs, were removed after 48, 96, and 120 hours of growth on methanol for determination of the hSOD and mRNA levels.

RNA was analyzed by Northern blots as described above. The three strains appeared to have similar amounts of SOD mRNA with each strain having more at the first time point, i.e. early during growth on methanol, than at the later two time points. The control strain had no mRNA which hybridized to the oligonucleotide probe. All three of the transformant strains possessed hSOD activity as measured by electrophoretic separation and assay (described in Example 4).

## Example 3

Growth of hSOD-expressing P. pastoris strains in 1-liter fermentors

Based on shake-flask expression results, two of the Mut⁺ strains, G + SOD104C10 (one copy) and G + SOD104C3 (two copies) and one of the Mut⁻ strains, G-SOD104C5, were evaluated in 1L fermentors for production of hSOD. The Mut⁺ cells were grown in a methanol-limited fed-batch mode, and the Mut⁻ cells were grown in both a methanol-excess fed-batch and a mixed-feed fed-batch mode as follows:

### A. Fermentation Modes

#### 1. Methanol-fed-batch, limited MeOH, Mut⁺ phenotypes

Run 441 G + SOD104C3 (double copy)
Run 442 G + SOD104C10 (single copy at HIS4)
Run 459 G + SOD104C10 (single copy at HIS4)
Run 460 G + SOD104C3 (double copy)

The fermentor was autoclaved with 700 ml minimal salts medium (final basal salts concentration of 3.3X) and 4% glycerol. After sterilization, 3 ml each of YTM4 and IM1 were added and the pH was brough to 5 with concentrated $NH_4OH$. Afterward, pH5 was maintained by addition of dilute (1:4) $NH_4OH$ containing 0.1% Struktol J673 antifoam (Struktol Co., Stow, OH). Inocula were prepared from selective plates and grown overnight at 30°C in phosphate-buffered 0.67% yeast nitrogen base (pH 5) containing 2% glycerol. The fermentor was inoculated with cultured cells and the batch growth regime lasted 18 to 24 hours. At the point of substrate exhaustion, a 50% glycerol feed (containing 12 ml/L each of YTM4 and IM1) was initiated at 12 ml/h for 7 h. At the point of substrate exhaustion, usually after six hours of glycerol feeding, an MeOH feed (containing 12 ml/L each of YTM4 and IM1) was initiated at 1.5 ml/hr and an additional 3 ml of YTM4 and IM1 were added to the fermentor. Adjustments in increments of 10% every 30 min were made over the course of the next 10 hours until a final feed rate of 7.5 ml/h was attained. The vessel was harvested 48-80 hours following MeOH induction.

#### 2. Limited MeOH, continuous culture, Mut⁺ phenotype

Run 476 G + SOD104C3 (double copy)

The fermentor was prepared as described for the limited MeOH-fed-batch protocol hereinabove. Upon glycerol exhaustion, a 5% MeOH feed (containing 4X Basal Salts, 12 ml YTM4 and IM1 per liter of MeOH) was initiated at 10 ml/h. The 5% MeOH feed was increased to 60 ml/h over the next six hours. Once the reactor liquid volume reached 1 liter (approximately 30 hours), a harvest stream was initiated at a rate equal to the feed rate to maintain a constant volume of 1 liter in the fermentor.

At 143 hours on MeOH, 1 ml of a 1% copper and zinc solution was added to the reactor. After 295 hours on MeOH, the feed and effluent rates were decreased to 30 ml/h. Continuous culture was switched to fed-batch mode after 431 hours by switching from the 5% MeOH feed to a 100% MeOH feed (plus 12 ml/L YTM4 and IM1) and terminating the effluent stream. The fed-batch mode was run as described above, for a period of 72 hours for a total of 503 hours of MeOH for the entire run.

### 3. Methanol-fed-batch, excess MeOH, Mut⁻ phenotypes

Run 445 G-SOD104C5 (single copy at AOX1)

The fermentor was prepared and inoculated as described above for a Mut$^+$ methanol-fed-batch run. At the point of substrate exhaustion a methanol feed (containing 12 ml/L each of YTM4 and IM1) was initiated at 0.5 ml/h and an additional 3 ml each of YTM4 and IM1 were added to the fermentor. The feed rate was increased over the course of the fermentation to maintain a residual MeOH concentration of approximately 4 g/l. The vessel was harvested from 6 to 10 days following MeOH induction.

### 4. Mixed-feed fed-batch, Mut⁻ phenotype

Run 446 G-SOD104C5 (single copy at AOX1)

The fermentor was prepared and inoculated as described above for Mut$^+$ methanol-fed-batch run. At the point of substrate exhaustion a 50% glycerol feed (containing 12 ml/L each of YTM4 and IM1) was initiated at 5.4 ml/h for 6 hours. Then, the glycerol feed rate was reduced to 3.6 ml/h, 3ml each of YTM4 and IM1 were added, and a MeOH feed (containing 12 ml/L each of YTM4 and IM1) was started. The initial rate of MeOH addition was about 1ml/h (MeOH:glycerol feed ratio of 0.7:1). Adjustments in increments of 10% every 30 minutes were made over the course of the next 10 hours until a final feed rate of 4.9 ml/h (MeOH:glycerol feed ratio of 2:1) was attained. The MeOH feed rate was adjusted so that the residual MeOH concentration did not exceed 4 g/l. The vessel was harvested 80 hours following MeOH induction.

## B. Fermentation Results

The time-course plots in Figure 2A show the build-up of cell density, measured as packed cell wet weight (WW) in Runs 441, 442, 445, and 446. The cell growth profiles were typical of those routinely observed in fermentation modes 1, 3 and 4 described above.

The Mut$^+$ strains from Runs 441 and 442 (double and single copy, respectively) attained high cell density (340-350 g WW/L) in only 54 hours, employing the methanol-fed- batch protocol (Fermentation Mode 1). The methanol-fed-batch protocol routinely used for Mut⁻ strains (Fermentation Mode 3) is a much longer fermentation, typically lasting from 6 to 10 days. This is illustrated by Run 445 which required 150 hours to attain 280 g WW/L cell density. Using a mixed-feed strategy in Run 446 (Fermentation Mode 3), it was demonstrated that the Mut⁻ strain G-SOD104C5 could be quickly grown to a density equivalent to the Mut$^+$ strains; 380 g WW/L (95 g dry weight/L) cell density was achieved in 80 hours.

The time-course of hSOD expression in Runs 441, 442, 445, and 446 (Figure 2B) indicates that in these Runs, the highest hSOD levels (3500 kU/L, or 0.92 g/L based on a specific activity of 3.8 U/μg) were produced by the double-copy strain G+SOD104C3. This strain also demonstrated one of the highest specific productivities, 240 U/g WW-h, as set forth in Table I.

Among the single-copy strains, expression level and specific productivity were highest in the Mut$^+$ strains, followed by the Mut⁻strains grown on a mixed substrate. The Mut⁻ cells grown in a methanol-limited fed-batch mode were less productive than the others.

As indicated by similar yields and specific productivities (Table I), Runs 441 and 442 were reproduced successfully in Runs 460 and 459, respectively. The latter two Runs were induced on methanol for 80 hours. Since the yield of hSOD is proportional to the amount of cell mass produced under inducing conditions, higher expression levels, 5100 kU/L and 1400 kU/L, respectively, were obtained in the longer runs (Figure 2C). Based on our determination of hSOD specific activity, the best level of expression was realized in Run 460. 1.3g hSOD was produced per liter of fermentor volume.

Table I

| hSOD-Expressing Pichia Strains | | | | | | | | | |
|------|--------|-----------|------|------------|------------|-----|---------------|--------------|--------------|
| RUN | STRAIN | PHENOTYPE | COPY | MAX SOD | MAX SOD | HRS | MAX Wet Wt. | SPECIFIC PRODUCTIVITY | VOLUMETRIC PRODUCTIVITY |
| | | | # | kU/L | g/L | | g/L | U/g/h | kU/L/h |
| 441 | G + SOD104C3 | Mut[+] | 2 | 3500 | 0.92 | 54 | 340 | 240 | 62 |
| 442 | G + SOD104C10 | Mut[+] | 1 | 1100 | 0.29 | 54 | 359 | 72 | 18 |
| 459 | G + SOD104C10 | Mut[+] | 1 | 1400 | 0.37 | 79 | 421 | 58 | 16 |
| 460 | G + SOD104C3 | Mut[+] | 2 | 5100 | 1.34 | 80 | 411 | 220 | 66 |
| 476[1] | G + SOD104C3 | Mut[+] | 2 | 640 | 0.17 | 431 | 80 | 330 | 38 |
| 476[2] | G + SOD104C3 | Mut[+] | 2 | 4600 | 1.21 | 503 | 350 | 204 | 55 |
| 445 | G + SOD104C5 | Mut[−] | 1 | 700 | 0.18 | 143 | 277 | 26 | 5.9 |
| 446 | G + SOD104C5 | Mut[−] | 1 | 920 | 0.24 | 71 | 382 | 50 | 13 |

[1] Run 476 was conducted first in a continuous culture mode, followed by a fed-batch mode. In the continuous culture mode hSOD levels were maintained for 431 hr and were insensitive to the addition of copper and zinc to the reactor

[2] Fed-batch mode of Run 476.

As hereinabove described, Run 476 was conducted first in a continuous culture mode, followed by a fed-batch mode. In the continuous culture, hSOD levels were maintained for 431 hours and were insensitive to the addition of copper and zinc to the reactor. As illustrated in Table I, the double-copy strain G + SOD104C3 fermented following this fermentation protocol gave the best specific productivity (330 U/g/h).

The above-discussed hSOD expression levels are based on data from activity assays disclosed in Example 4 hereinbelow. Since activity assays can only measure functional molecules, denatured or inactive enzymes would escape detection. Therefore, the indicated expression levels are lower limits; the true levels are equal to or greater than these values.

Example 4

Characterization of hSOD

A. Activity Assays

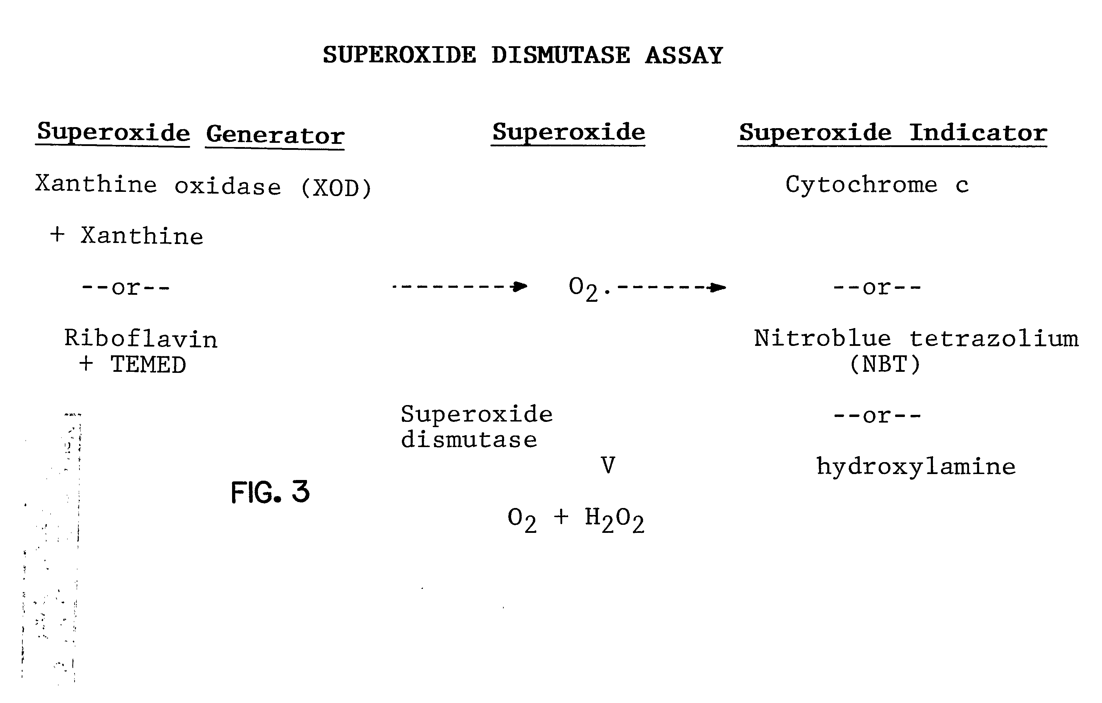

(The principle of SOD activity assays is illustrated in Figure 3)

1. Nitrite Assay

The nitrite assay [Y. Oyanagui, Anal. Biochem. 142, 290 (1984)] was used to measure hSOD activity. This assay is based on the oxidation of hydroxylamine by hSOD to nitrite that is then detected with an indicator dye.

The nitrite assay has proved to be particularly successful for measuring hSOD concentrations in Pichia lysates. Table II shows that several samples collected during fermentation runs give high activity, whereas the control sample (G-PAO804) yields a much lower value, often as little as 5% of the recombinant strains. Thus, this assay can differentiate recombinant hSOD activity from other SOD-like activities in the lysate and, as shown in Table II, with a deviation generally less than 10%.

Table II

| SOD Assay Variation | | |
|---|---|---|
| Assay Sample | Activity[1] | % Deviation[2] |
| (Run #-hours) | (units/μl) | |
| 441-21 | .791 | 10.44 |
| 441-54 | 1.779 | 4.00 |
| 442-21 | .385 | 6.94 |
| 442-54 | .569 | 11.40 |
| 445-34 | .340 | 6.89 |
| 445-143 | .442 | 6.85 |
| 804 (control) | .089 | 17.10 |

[1]Numbers represent the mean of 4 assay values.
[2] Calculated from the sample standard deviation of the mean.

The nitrite assay was standardized to the cytochrome c assay by testing both a human erythrocyte SOD standard (Sigma Chemical Company) and human recombinant SOD purified from P. pastoris in each of the assays. The erythrocyte SOD was reported by the manufacturer to have a specific activity of 2.7 units/μg in the cytochrome c assay. We have measured a specific activity of 3.8 units/μg in this assay and found that the purified recombinant hSOD prepared according to the invention, yielded the same value. All protein concentrations were verified using quantitative amino acid analysis. The nitrite assay also gave identical values for the two preparations. Therefore, the simpler and more discriminating nitrite assay can be used to assay lysate samples if the values obtained from unknowns are always compared with those obtained from pure enzyme of known specific activity.

## 2. Electrophoretic separation and assay

The endogenous and heterologous hSOD activites in P. pastoris can be separated and assayed in an acrylamide gel following non-denaturing electrophoresis. For this assay the most convenient superoxide generator and detector are, respectively, riboflavin/TEMED and nitroblue tetrazolium (NBT) Beauchamp et al., Anal. Biochem. 44, 276 (1971)]. NBT is converted to the blue product throughout the gel except in areas where SOD is present to scavenge the superoxide. Extracts from transformed cells grown in shake flasks were separated by electrophoresis and found to have SOD activity that comigrates with the human standard. Control cell extracts also produced a zone of clearing on the gels, but this was in a region of the gel well isolated from the position of the human form of SOD, at a higher electrophoretic mobility. Surprisingly, transformed cells that express maximum levels of human SOD did not produce any noticeable activity at the same gel position as the control cells. There was, however, a new zone of clearing that appeared at an intermediate mobility between the position of endogenous P. pastoris SOD and recombinant human SOD. This suggests that a heterodimer may be formed between the endogenous P. pastoris SOD and the recombinant human SOD.

In a typical electrophoretic separation and assay, aliquots equivalent to 4 μl of extracts in 50 mM phosphate, pH 7.8, were submitted to non-denaturing electrophoresis, and superoxide radicals were generated and detected as described above.

## B. Immunodetection

Goat antiserum to authentic hSOD was used. Rabbit antigoat IgG antiserum (Organon Teknika, Westchester, PA) was used as a second antibody to increase the binding efficiency of the $^{125}$I-Protein A.

SDS-polyacrylamide gel electrophoresis was used to separate hSOD from the cross-reactive components in P. pastoris lysates. Samples were reduced, denatured and submitted to electrophoresis on 15% acrylamide gels to resolve the 16 kilodalton hSOD monomers. Lysates from SOD transformants grown in shake flask had both an immunoreactive band that comigrated with the hSOD standard and cross-reactive material of higher apparent molecular weight. Control lysates, on the other hand, had very little immunoreactivity in the hSOD region of the gel, but showed the same high molecular weight material. When the shake flask lysates were compared, the two-copy Mut$^+$ strain (G + SOD104C3) consistently gave a more intense band that any of the single-copy strains.

Western blots can provide a quantitative measure of the amount of immunoreactive protein in a lysate by careful comparison, on the same gel, with several concentrations of standard. The results from quantitative Western blots confirmed and complemented the results from the nitrite spectrophotometric assay and the NBT activity gels. The measured amount of hSOD activity and hSOD protein was the same within experimental error. This is an important comparison since it suggests that all of the hSOD polypeptides are properly folded and fully active. In addition, Coomassie Blue staining of either the denaturing or native gels showed clearly visible bands that comigrate with hSOD standard. These Coomassie bands were of an intensity expected according to the other estimates of total hSOD protein in the sample. Since the denaturing gels separate according to size and the native gels separate on the basis of size and charge, the fact that the recombinant hSOD comigrated with the standard in both systems is evidence that the recombinant enzyme produced in Pichia is properly folded, is the correct size, and displays the proper surface charge.


Example 5


Purification and stability of hSOD produced in P. pastoris

Greater than 90% of the recombinant hSOD was extracted from P. pastoris cells lysed with glass beads in the presence of simple buffers. Typically, a 50 mM sodium or potassium phosphate buffer containing 0.1 mM EDTA at pH 7.8 was used, and recovery, after corrections for pellet volume, was at least 95 % when measured by either activity assay or Western blots. (Any buffer in which the enzyme is stable may be used.) Recombinant hSOD was purified from the cell lysate essentially as described by McCord et al., J. Biol. Chem. 241, 6049 (1969) for erythrocyte lysates.

Hemoglobin is precipitated from the erythrocyte preparation by an ethanol-chloroform treatment. Likewise, many yeast proteins were removed from the P. pastoris lysate. Ethanol, 25 % of the lysate volume, was added dropwise to the stirred lysate suspension that was kept below 4°C in a salt water-ice bath. Chloroform was added in a similar manner (15 percent of the initial lysate volume). Contaminating proteins were allowed to denature and precipitate by stirring for an additional 15 minutes in the ice bath, and the precipitate was removed by centrifugation.

Superoxide dismutase was further purified by a phase extraction step. Dibasic potassium phosphate was added to the supernatant that was warmed to 25°C. When thirty percent $K_2HPO_4$ (weight-to-volume) was dissolved in the ethanol-chloroform solution, hSOD partitioned into the upper phase away from the lower, high salt phase. The upper phase was cooled to 4°C and clarified by centrifugation.

Recombinant hSOD was concentrated by precipitating with cold acetone (75% of the supernatant volume), centrifuged, and resuspended in a reduced volume. The solution was dialyzed to equilibrium against 2.5 mM potassium phosphate, pH 7.8. (In place of potassium phosphate buffer any low ionic strength buffer around a neutral pH could be used.) This buffer should be the equilibration buffer for DE-52, or any other similar anion exchanger used in the next step. hSOD eluted from DE-52 in a gradient of 2.5 to 100 mM potassium phosphate, pH 7.8.

When stored as a lysate at 3-5°C, the hSOD activity is stable for at least four weeks. As a purified protein, recombinant hSOD from P. pastoris is stable for at least eight weeks at 3-5°C.


## Claims

1. A P. pastoris cell containing in its genome at least one copy of a DNA sequence operably encoding in P. pastoris a polypeptide having superoxide dismutase (SOD) activity under the regulation of a promoter region of a P. pastoris gene.

2. A P. pastoris cell as claimed in claim 1, wherein the P. pastoris gene is the P. pastoris AOXI gene.

3. A P. pastoris cell as claimed in claim 2 which contains at least two copies of said DNA sequence.

4. A P. pastoris cell as claimed in claim 1 which containing in its genome at least one copy of an expression cassette, comprising in the direction of transcription, a promoter region of a first P. pastoris gene, a DNA sequence encoding in P. pastoris a polypeptide having SOD activity and a transcription terminator of a second P. pastoris gene, the first and second P. pastoris genes being identical or different, and the segments of the expression cassette being in operational association.

5. A P. pastoris cell as claimed in claim 4 wherein the first and second P. pastoris genes are identical and are the P. pastoris AOXI gene.

6. A P. pastoris cell as claimed in claim 5 containing at least two copies of the expression cassette.

7. A P. pastoris cell as claimed in claim 5 containing a single copy of the expression cassette integrated by replacement at the AOXI locus of the P. pastoris genome.

8. A P. pastoris cell as claimed in claim 5 containing a single copy of the expression cassette integrated by addition at the HIS4 locus of the P. pastoris genome.

9. A P. pastoris cell as claimed in claim 5 containing a single copy of the expression cassette integrated by addition at the AOX1 locus of the P. pastoris genome.

10. A P. pastoris cell as claimed in claim 6 containing two copies of the expression cassette integrated by addition at the AOX1 and HIS4 loci of the P. pastoris genome, respectively.

11. A P. pastoris cell as claimed in any one of the preceding claims which is a cell of the P. pastoris host strain GS115 (ATCC 20864).

12. A P. pastoris cell as claimed in any one of claims 1 to 10 wherein the DNA sequence operably encodes human Cu/Zn superoxide dismutase (hSOD) in P. pastoris.

13. A P. pastoris cell as claimed in claim 11 wherein the DNA sequence operably encodes hSOD in P. pastoris.

14. A DNA fragment optionally contained within or which is a circular plasmid comprising at least one copy of an expression cassette comprising in the direction of transcription, a promoter region of a first P. pastoris gene, a DNA sequence encoding in P. pastoris a polypeptide having SOD activity and a transcription terminator of a second P. pastoris gene, the first and second P. pastoris genes being identical or different, and the segments of the expression cassette being in operational association.

15. A DNA fragment as claimed in claim 14 wherein the first and second P. pastoris genes are identical and are the P. pastoris AOXI gene.

16. A DNA fragment as claimed in claim 14 or claim 15 further comprising a selectable marker gene and ends having sufficient homology with a target gene to effect integration of the DNA fragment therein.

17. A DNA fragment as claimed in claim 16 wherein the target gene is the P. pastoris AOXI gene.

18. A DNA fragment as claimed in claim 16 or claim 17 wherein the selectable marker gene is the P. pastoris HIS4 gene.

19. A DNA fragment as claimed in any one of claims 14 to 18 wherein the DNA sequence operably encodes hSOD in P. pastoris.

20. A DNA fragment as claimed in claim 19 which is a BgIII digest of the plasmid pSOD104.

21. A DNA fragment as claimed in claim 15, further comprising a selectable marker gene and at least one additional DNA sequence having sufficient homology with a target gene to effect integration thereto.

22. A DNA fragment as claimed in claim 21, wherein the target gene is the P. pastoris AOXI gene or the P. pastoris HIS4 gene.

23. A DNA fragment as claimed in claim 21 or claim 22, wherein the selectable market gene is the P. pastoris HIS4 gene.

24. A DNA fragment as claimed in any one of claims 21 to claim 23, wherein the DNA sequence operably encodes hSOD in P. pastoris.

25. A DNA fragment as claimed in claim 24 which is the plasmid pSOD104.

26. An expression vector containing at least one copy of an expression cassette comprising in the direction of transcription, a promoter region of a first P. pastoris gene, a DNA sequence encoding in P. pastoris a polypeptide having SOD activity and a transcription terminator of a second P. pastoris gene, the first and second P. pastoris genes being identical or different, and the segments of the expression cassette being in operational association.

27. An expression vector as claimed in claim 26 containing a DNA fragment as claimed in any one of claims 15 to 19.

28. An expression vector as claimed in claim 26 or claim 27 further comprising sequences allowing for its replication and selection in bacteria.

29. An expression vector as claimed in claim 28, which is a pBR322 derivative.

30. A culture of viable P. pastoris cells as claimed in any one of claims 1 to 13.

31. A process for producing polypeptides having SOD activity comprising growing P. pastoris transformants containing in their genome at least one copy of a DNA sequence operably encoding in P. pastoris a polypeptide having SOD activity in operational association with a promoter region of a P. pastoris gene,

under conditions allowing the expression of the DNA sequence in the P. pastoris.

32. A process as claimed in claim 31 wherein the P. pastoris transformants contain in their genome at least two copies of the DNA sequences.

33. A process as claimed in claim 31 wherein the P. pastoris transformants contain in their genome at least one copy of an expression cassette, comprising in the direction of transcription, a promoter region of a first P. pastoris gene, a DNA sequence encoding in P. pastoris a polypeptide having SOD activity and a transcription terminator of a second P. pastoris gene, the first and second P. pastoris genes being identical or different, and the segments of the expression cassette being in operational association.

34. A process as claimed in claim 33 wherein the transformants are obtained by transformation with a DNA fragment as claimed in any one of claims 14 to 25.

35. A process as claimed in claim 34 wherein the transformants are grown in a medium containing methanol as a carbon source.

36. A process as claimed in any one of claims 4 to 16, wherein the transformants are developed from the P. pastoris his4⁻ strain GS115.

37. A process as claimed in any one of claims 4 to 16 wherein the transformants have the Mut⁻ phenotype.

38. A process as claimed in any one of claims 4 to 16 wherein the transformants have the Mut⁺ phenotype.

39. A process as claimed in any one of claims 4 to 16 further comprising the step of harvesting the SOD from the transformants.

Claims for the following Contracting States: ES, GR

1. A process for producing polypeptides having SOD activity comprising growing P. pastoris transformants containing in their genome at least one copy of DNA sequence operably encoding in P. pastoris a polypeptide having SOD activity in operational association with a promoter region of a P. pastoris gene, under conditions allowing the expression of the DNA sequence in the P. pastoris.

2. A process as claimed in claim 1 wherein the P. pastoris transformants contain in their genome at least two copies of the DNA sequences.

3. A process as claimed in claim 2 wherein the P. pastoris transformants contain in their genome at least one copy of an expression cassette, comprising in the direction of transcription, a promoter region of a first P. pastoris gene, a DNA sequence encoding in P. pastoris a polypeptide having SOD activity and a transcription terminator of a second P. pastoris gene, the first and second P. pastoris genes being identical or different, and the segments of the expression cassette being in operational association.

4. A process as claimed in claim 3 wherein said transformants are obtained by transformation with a DNA fragment optionally contained within or which is a circular plasmid comprising at least one copy of an expression cassette comprising in the direction of transcription, a promoter region of a first P. pastoris gene, a DNA sequence encoding in P. pastoris a polypeptide having SOD acitivity and a transcription teminator of a second P. pastoris gene, the first and second P. pastoris genes being identical or different, and the segments of the expression cassette being in operational association.

5. A process as claimed in claim 4 wherein in the DNA fragment the first and second P. pastoris genes are identical and are the P. pastoris AOXI gene.

6. A process as claimed in claim 4 or claim 5 wherein the DNA fragment further comprises a selectable marker gene and ends having sufficient homology with a target gene to effect integration of the DNA fragment therein.

7. A process as claimed in claim 6 wherein the target gene is the P. pastoris AOXI gene.

8. A process as claimed in claim 6 or claim 7 wherein the selectable marker gene is the P. pastoris HIS4 gene.

9. A process as claimed in any one of claims 4 to 8 wherein the DNA sequence of the DNA fragment operably encodes hSOD in P. pastoris.

10. A process as claimed in claim 9 wherein the DNA fragment is a BglII digest of the plasmid pSOD104.

11. A process as claimed in claim 5 wherein the DNA fragment further comprises a selectable marker gene and at least one additional DNA sequence having sufficient homology with a target gene to effect integration thereto.

12. A process as claimed in claim 11 wherein the target gene is the P. pastoris AOXI gene or the P. pastoris HIS4 gene.

13. A process as claimed in claim 11 or claim 12 wherein the selectable marker gene is the P. pastoris HIS4 gene.

14. A process as claimed in any one of claims 11 to 13 wherein the DNA sequence operably encodes hSOD in P. pastoris.

15. A process as claimed in claim 14 wherein the DNA fragment is the plasmid pSOD104.

16. A process as claimed in any one of claims 4 to 15 wherein the transformants are grown in a medium containing methanol as a carbon source.

17. A process as claimed in any one of claims 4 to 16 wherein the transformants are developed from the P. pastoris his4⁻ strain GS115.

18. A process as claimed in any one of claims 4 to 16 wherein the transformants have the Mut⁻ phenotype.

19. A process as claimed in any one of claims 4 to 16 wherein the transformants have the Mut⁺ phenotype.

20. A process as claimed in any one of claims 4 to 16 further comprising the step of harvesting the SOD from the transformants.

```
     ggatccatggcgacgaaggccgtgtgcgtgctgaagggcgacggcccagtgcagggcatc
  1  ----------+----------+----------+----------+----------+--------+ 60
     cctaggtaccgctgcttccggcacacgcacgacttcccgctgccgggtcacgtcccgtag

     GlySerMetAlaThrLysAlaValCysValLeuLysGlyAspGlyProValGlnGlyIle -


     atcaatttcgagcagaaggaaagtaatggaccagtgaaggtgtggggaagcattaaagga
 61  ----------+----------+----------+----------+----------+--------+ 120
     tagttaaagctcgtcttcctttcattacctggtcacttccacacccccttcgtaatttcct

     IleAsnPheGluGlnLysGluSerAsnGlyProValLysValTrpGlySerIleLysGly -
```

## FIG. 1−1

EP 0 388 008 A1

```
      ctgactgaaggcctgcatggattccatgttcatgagtttggagataatacggcaggctgt
121   ----------+---------+---------+---------+---------+---------+ 180
      gactgacttccggacgtacctaaggtacaagtactcaaacctctattatgccgtccgaca

      LeuThrGluGlyLeuHisGlyPheHisValHisGluPheGlyAspAsnThrAlaGlyCys -


      accagtgcaggtcctcactttaatcctctatccagaaaacacggtgggccaaaggatgaa
181   ----------+---------+---------+---------+---------+---------+ 240
      tggtcacgtccaggagtgaaattaggagataggtcttttgtgccacccggtttcctactt

      ThrSerAlaGlyProHisPheAsnProLeuSerArgLysHisGlyGlyProLysAspGlu -
```

## FIG. 1-2

EP 0 388 008 A1

```
     gagaggcatgttggagacttgggcaatgtgactgctgacaaagatggtgtggccgatgtg
241  ----------+---------+---------+---------+---------+---------+ 300
     ctctccgtacaacctctgacccgttacactgacgactgtttctaccacaccggctacac

     GluArgHisValGlyAspLeuGlyAsnValThrAlaAspLysAspGlyValAlaAspVal -


     tctattgaagattctgtgatctcactctcaggagaccattgcatcattggccgcacactg
301  ----------+---------+---------+---------+---------+---------+ 360
     agataacttctaagacactagagtgagagtcctctggtaacgtagtaaccggcgtgtgac

     SerIleGluAspSerValIleSerLeuSerGlyAspHisCysIleIleGlyArgThrLeu -
```

<div align="center">FIG. 1−3</div>

EP 0 388 008 A1

```
       gtggtccatgaaaaagcagatgacttgggcaaaggtggaaatgaagaaagtacaaagaca
361    ----------+---------+---------+---------+---------+---------+ 420
       caccaggtactttttcgtctactgacccgtttccacctttacttctttcatgtttctgt

       ValValHisGluLysAlaAspAspLeuGlyLysGlyGlyAsnGluGluSerThrLysThr -


       ggaaacgctggaagtcgtttggcttgtggtgtaattgggatcgcccaatagtaactgcag
421    ---------+---------+---------+---------+---------+---------+ 480
       cctttgcgaccttcagcaaaccgaacaccacattaaccctagcgggttattattgacgtc

       GlyAsnAlaGlySerArgLeuAlaCysGlyValIleGlyIleAlaGlnEndEndLeuGln -
```

EP 0 388 008 A1

FIG. 1−4

FIG. 2-A

FIG. 2-B

FIG. 2-C

# SUPEROXIDE DISMUTASE ASSAY

**Superoxide Generator**   **Superoxide**   **Superoxide Indicator**

Xanthine oxidase (XOD)                     Cytochrome c

  + Xanthine

    --or--    $- - - - - \rightarrow$  $O_2 . - - - - \rightarrow$    --or--

  Riboflavin                               Nitroblue tetrazolium
  + TEMED                                         (NBT)

                    Superoxide                 --or--
                    dismutase
                              V           hydroxylamine

                    $O_2 + H_2O_2$

FIG. 3

EP 0 388 008 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP-A-0 226 846 (PHILLIPS PETROLEUM COMPANY) * the whole document * | 1-39 | C 12 N 15/53<br>C 12 N 15/81 //<br>(C 12 N 15/81<br>C 12 R 1:84 ) |
| D,Y | EP-A-0 138 111 (CHIRON CORPORATION) * the whole document * | 1-39 | |
| A | EP-A-0 256 421 (PHILLIPS PETROLEUM COMPANY) * the whole document * | 1,14,26 ,30,31 | |
| D,Y | EP-A-0 183 071 (PHILLIPS PETROLEUM COMPANY) * the whole document * | 1-39 | |
| Y | EP-A-0 283 244 (CHIRON CORPORATION) * the whole document * | 1-39 | |
| D,A | EP-A-0 188 677 (PHILLIPS PETROLEUM COMPANY) * the whole document, in particular page 11, line 3 - page 12, line 22; claims 11,23,27-28 * | 1,14,26 ,30,31 | **TECHNICAL FIELDS SEARCHED (Int. Cl.5)**<br>C 12 N 15/53<br>C 12 N 15/81 |
| D,A | EP-A-0 226 752 (PHILLIPS PETROLEUM COMPANY) * the whole document, in particular page 9, line 35 - page 10, line 6 * | 1,14,26 ,30,31 | |

The present search report has been drawn up for all claims

| Place of search | Date of completion of the search | Examiner |
|---|---|---|
| BERLIN | 18-06-1990 | JULIA P. |